Europäisches Patentamt

European Patent Office

Office européen des brevets

(19)

(11) EP 0 842 729 A1

(12) EUROPEAN PATENT APPLICATION

(43) Date of publication:
20.05.1998   Bulletin 1998/21

(51) Int Cl.⁶: **B23K 26/00**, A61F 2/06

(21) Application number: 97308358.7

(22) Date of filing: 21.10.1997

(84) Designated Contracting States:
AT BE CH DE DK ES FI FR GB GR IE IT LI LU MC NL PT SE
Designated Extension States:
AL LT LV RO SI

(30) Priority: 21.10.1996 US 732767

(71) Applicants:
• Arterial Vascular Engineering, Inc.
  Santa Rosa, California 95403 (US)
• THE REGENTS OF THE UNIVERSITY OF CALIFORNIA
  Oakland, CA 94612-3550 (US)

(72) Inventors:
• Lashinski, Randall T.
  Santa Rosa, California 95403 (US)
• Birdsall, Matthew J.
  Santa Rosa, California 95405 (US)
• Perry, Michael D.
  Livermore, California 94550 (US)
• Stuart, Brent C.
  Fremont, California 94536 (US)
• Nguyen, Hoang T.
  Livermore, California 94550 (US)

(74) Representative: Bayliss, Geoffrey Cyril et al
  BOULT WADE TENNANT,
  27 Furnival Street
  London EC4A 1PQ (GB)

(54) **Method and apparatus for laser processing of intravascular devices**

(57) A method of forming an intravascular device, such as a stent, without creating shock or thermal stress affected areas to or in the final product, employs a laser (20) capable of producing very short pulses. In order to remove a volume of material from a workpiece (40) to create the intravascular device, laser pulses of less than 100 picoseconds in duration are directed at the area of the workpiece to be removed. By providing laser pulses with pulse durations such that the thermal penetration depth during the pulse is on the order of the optical penetration depth, very small amounts of material can be removed per laser pulse with negligible transport of energy by shock or thermal conduction away from the volume of interest. This creates an intravascular device that does not have features which have been subjected to shock or thermal stress, improving the integrity of the device.

Figure 2

## Description

The present invention is related to the field of intravascular devices, and more particularly, to the manufacture of high precision intravascular devices, such as stents, using a laser system in the manufacturing process.

Laser interaction with dielectrics, semiconductors and metals has been studied for over thirty-five years for applications as diverse as materials processing, inertial confinement fusion, and recently surgery. Laser processing of material (cutting, welding, peening, etc.) is well established. To date, materials processing has been limited to two basic physical mechanism: thermal or photodissociative.

The thermal mechanism is by far the more common of the two mechanisms. In this case, the basic interaction is the deposition of energy from the incident laser beam in the material of interest in the form of heat (lattice vibrations). Absorption of laser energy by the material may differ strongly between materials (e.g., resistive heating of conducting electrons in metals, electronic or vibronic absorption in dielectrics). Laser absorption may also be strongly wavelength dependent particularly in the case of dielectrics (depending on the presence of an absorption band at the laser wavelength) or semiconductors (location of the band gap relative to the energy of the laser photon). However, regardless of the detailed mechanism of absorption of laser energy, the deposited energy results in simple heating of the material. The laser energy that is absorbed results in a temperature increase at and surrounding the absorption site. As the temperature increases to the melting or boiling point, material is removed by conventional melting or vaporization. Depending on the pulse duration of the laser, the temperature rise in the irradiated zone may be very fast resulting in thermal ablation and shock. In this case, the irradiated area experiences a temperature rise to near or above the boiling point faster than heat can be conducted into the bulk material. The irradiated zone may be vaporized or simple ablate off due to the fact that the local thermal stress has become larger than the yield strength of the material (thermal shock).

In all these cases, where material is removed via a thermal mechanism, there is an impact on the material surrounding the site where material has been removed. The surrounding material will have experienced a large temperature excursion or shock often resulting in significant collateral damage. This affected zone may range from a few microns to several millimeters depending on the material type, laser pulse duration and other factors (e.g., active cooling). In many low precision applications, the presence of the affected zone is mot limiting. As a result, lasers are routinely used to cut and weld metals in heavy industry (e.g., auto manufacture). In high precision applications, the presence of the heat or shock affected zone may be severely limiting. Devices with features on the order of a few tens of microns cannot tolerate stress induced in the material during the machining process. Even the slightest thermal stress or shock can destroy the feature of interest. Such devices include many used in the medical field, including stents.

Another limitation of conventional laser processing in high precision applications is the presence of redeposited or resolidified material. As mentioned previously, laser cutting or drillling occurs by either melting or vaporizing the material of interest. The surface adjacent to the removed area will have experienced significant thermal loading, often resulting in melting. This melting can be accompanied by flow prior to solidification. This can result in the deposition of slag surrounding the kerf.

In many high precision applications, the presence of slag is unacceptable. In the cases where the deposition of conventional slag can be prevented, redeposition of vaporized material on the walls or upper surface of the kerf is common. This condensate often reduces the quality of the cut and decreases the cutting efficiency since the laser must again remove this condensate before interacting with the bulk material underneath. Many of these limitations of laser cutting by the thermal mechanism can be reduced somewhat by the use of secondary techniques during the cutting process. The most common of these techniques are active cooling of the material of interest either during or immediately following the laser pulse, and the use of high pressure gas jets to remove vaporized or molten material from the vicinity of the cut to prevent redeposition. These techniques can be effective at improving the kerf at the cost of a significant increase in system complexity and often a decrease in cutting efficiency.

An example of a system for laser cutting a stent is described in U.S. Patent 5,073,694, in which a complicated arrangement is provided for supplying coolant during the laser processing to the interior of the hollow metal tube forming the stent. The dross which forms during cutting drops into the coolant, solidifies and is flushed out of the workpiece along with the coolant. This system suffers from added complexity and the decrease in cutting efficiency noted above.

Another drawback to the thermal mechanism for cutting with conventional laser such as CO2, copper vapor, or ND:YAG is the low cutting efficiency or high volumetric energy deposition required. This parameter refers to the total amount of laser energy required to remove a given volume of material (Joules/cm3). The minimum volumetric energy deposition is determined simply by the heat capacity and boiling point of the material of interest,

$$U_{min} = C\int pdT$$

where the limits of the integrand run from the initial temperature, $T_o$ to the boiling point, $T_b$. In the case of conventional thermal cutting, the energy required to remove a given volume of material, U, is much larger than this minimum value due to the energy lost from the volume of interest to conduction and shock. The cutting efficiency, $\eta=U_{min}/U$, is often quite low (0.1-a few percent) with conventional lasers. For the case of cutting thin (<1 mm thick) metals, improvements in cutting efficiency can be achieved by operating the laser to achieve shock cutting. In this technique, one takes advantage of the shock wave produced by an intense laser pulse by adjusting laser parameters such that the induced shock wave produces a stress in the region of interest which is beyond the yield strength of the material. The material then blows out the back surface. This technique is applicable only to processing thin materials and requires high laser pulse energy.

The second mechanism, photodissociation, has found the greatest success in the processing of organic materials (e.g., polymers, soft tissue) in both medical and industrial applications. In this mechanism, the photon energy is sufficiently high to dissociate (break) a molecular bond of interest. Ideally, the resulting molecular or atomic fragments are volatile and leave the surface. Extremely high precision cutting and drilling can be achieved with this mechanism in materials which exhibit high absorption of the laser wavelength. Photo-keratotomy and corneal sculpting are high visibility medical applications. And microdrilling of polymers for ink jets is a well-established industrial application of this laser cutting technique. Since photodissociation often requires very energetic photons, the laser must produce ultraviolet light. The most common laser sources are excimer [ArF (193 nm), KrF (248nm) or XeCl (308 nm)] based systems. These ultraviolet lasers require complex and sophisticated delivery and support systems to minimize the extreme personnel hazards associated with intense ultraviolet light and reactive gases (fluorine and chlorine), and thus have their own drawbacks.

There is a need for a method of laser cutting an intravascular device, which requires high precision in manufacture, that avoids the addition of shock or thermal stress to the intravascular devices without requiring any cooling of the workpiece during laser processing.

This and other needs are met by the present invention which provides a method of laser processing an intravascular device, such as a stent, utilizing a laser that provides very short laser pulses (e.g., 100 picoseconds). The use of such a laser, in accordance with an embodiment of the method of the present invention, allows very small amounts of material (0.01-1 micron) to be removed per laser pulse with extremely small transport of energy either by shock or thermal conduction away from the volume of interest. This offers extremely high precision machining with no heat or shock affected zone to or in the final product.

The earlier stated needs are met by an embodiment of the present invention which provides a method of forming an intravascular device, comprising the steps of positioning a workpiece for exposure to laser pulses, and subjecting the workpiece to laser pulses, each laser pulse having a duration of less than approximately 100 picoseconds, to remove material from the workpiece to form the intravascular device.

Another embodiment of the present invention provides a method of manufacturing an intravascular device, comprising the steps of positioning a workpiece for exposure to a laser beam, and directing a laser beam at the workpiece to remove at least one volume of material from a site on the workpiece with minimal transport of energy to the areas of the workpiece surrounding the volume of material.

The method of forming an intravascular device according to the present invention has a number of different advantages. The processing efficiency is increased, since complicated cooling and dross removal systems are not necessary. Also, cutting efficiency with short pulses is significantly higher than for conventional long pulse lasers because there is little loss of energy away from the region of interest since thermal conduction during the pulse is negligible, and since there is no vaporization or transport of material during the pulse. In addition, the intravascular device that is formed will not contain thermal or shock affected areas due to the negligible energy transport during the laser pulse. This is especially critical in intravascular devices, in which the features of interest may be extremely small, on the order of a few tehs of microns.

The earlier stated needs are also met by an embodiment of the present invention which provides an intravascular device comprising material areas forming a pattern of material, and spaces from which volumes of material have been removed from the intravascular device, the spaces being formed by directing laser pulses having a pulse duration lass than 100 picoseconds at the intravascular device to remove the material and form the pattern of material without transferring significant energy to the material areas forming the pattern of material.

The intravascular device of the present invention exhibits greater structural integrity because it does not have areas that have been subjected to thermal stress, shock and/or degradation, since only pulses of extremely short duration have been used to remove the material from the workpiece. Hence, an extremely high precision intravascular device, such as a stent, with an improved structural integrity, is provided by the present invention.

The foregoing and other features, aspects and advantages of the present invention will become more apparent from the following detailed description of the present invention when taken in conjunction with the accompanying drawings.

Figure 1a is a perspective view of a workpiece prior to laser processing according to an embodiment of the present invention.

Figure 1b is a perspective view of a stent that is formed in accordance with an embodiment of the present invention.

Figure 2 is a schematic depiction of a laser system for cutting a workpiece in accordance with an embodiment of the method of the present invention.

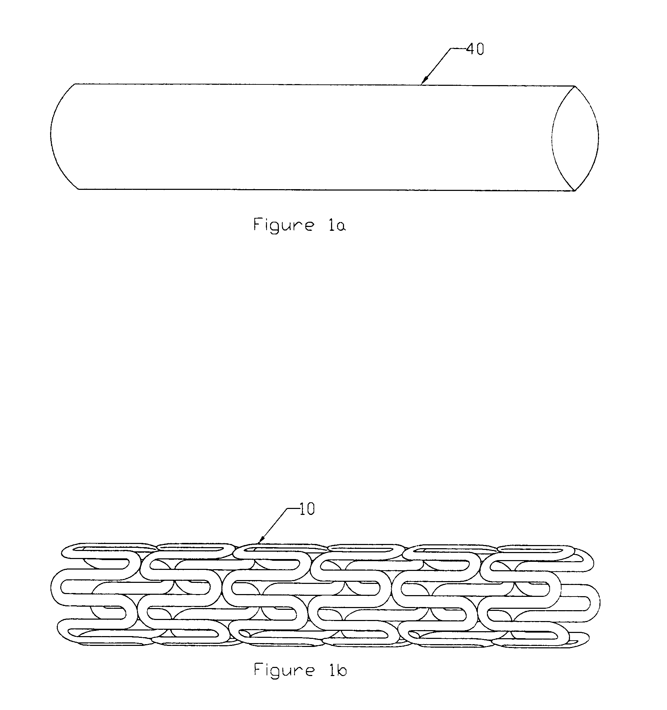

An example of a stent that has been cut from a workpiece in accordance with an embodiment of the present invention is depicted in Figure 1b. The workpiece 40 prior to cutting is depicted in Figure 1a. A stent is a high precision intravascular device, formed as a metal prosthesis designed to hold open passageways in the body. For implantable devices, the workpiece is made of any suitable material, such as stainless steel, shape-memory alloys, polymeric implantation material, platinum-iridium, etc. Other materials may be used when the implantable device is not intended for long term implantation or use within the body, such as diagnostic or short term implantation devices.

Stents are minimally invasive treatment devices which can be used in combination with balloon angioplasty or artherectomy procedures in the treatment of artherosclerosis. Despite the popularity of balloon angioplasty, a significant percentage of balloon angioplasty patients suffer a significant re-narrowing of the treated blood vessel within six months following initial treatment, a condition known as restenosis. The use of stents in conjunction with balloon angioplasty indicates restenosis rates well below those observed when stents are not used.

Stents may have a design that provides a number of technical features that enhance the use of the stents in the human body. For example, it is desirable to provide a stent with flexibility to allow access to tortuous or curved vessels. Also, high radial strength and axial stability are desired to optimize diameter post procedure.

Stents are available in a variety of basic designs and sizes. Some of the basic designs of stents include tubular-slotted, wire-like, wire-mesh, and others known to those of ordinary skill in the art. Stents, such as stent 10 of Figure 1b, have dimensions typically in the millimeter range. For example, the length of the stent 10 in Figure 1a may be approximately 18 mm, the outer diameter approximately 3.5 to 4 mm, with a wall thickness of approximately 0.002 to 0.004 inches. These dimensions and the structure of the depicted embodiment are exemplary only, however, as other embodiments of stents formed according to the present invention can have different sizes and configurations.

In the laser processing of the workpiece, for example, a hollow tube, flat sheet, or wire formation. etc., is presented to the laser. The laser, typically under the guidance of a computer, makes the cuts or other processing necessary to form the intricate structure to provide the design features of the stent, or other intravascular device. The main concern created by laser processing of intravascular devices is the shock and thermal stress generated in the intravascular device by the laser during the cutting. Although measures may be taken to cool the workpiece during cutting, such systems are cumbersome and relatively ineffective in reducing shock or thermal stress.

The method of processing a workpiece with a laser to form an intravascular device according to embodiments of the present invention uses very short (<100 picoseconds) laser pulses to cut with, in comparison to prior art laser cutting methods. Before describing an exemplary laser system that can be used to provide laser cutting with very short pulses, the principles and advantages of such laser cutting will be discussed.

In dielectric materials which are otherwise transparent to the laser wavelength, free electrons are produced by multiphoton absorption. These free electrons are accelerated in the laser field and produce additional ionization in the dielectric by collisions. A critical density plasma is formed within a fraction of a micron of the surface. This plasma expands from the surface with an initial temperature of 1-1000 eV (dependent upon laser intensity and wavelength). Very little energy (either by shock or by thermal conduction) is coupled into the bulk material since the laser pulse duration is adjusted to be below the characteristic time for kinetic energy transfer from the electrons to the lattice. A typical characteristic time is approximately 10 picoseconds for common dielectrics (e.g., $SiO_2$).

In metals, although the absorption mechanism for the laser energy is the same as in the case of long pulse lasers, the short (<100 psec) duration offers a simple and striking advantage. By adjusting the pulse duration such that the thermal penetration depth during the pulse, $L_{th}=2\sqrt{\alpha\tau}$, is on the order of the optical penetration depth, $\delta= [2/(\sigma\omega\mu_0)]^{\frac{1}{2}}$, very small amounts of material (0.01-1 micron) can be removed per laser pulse with extremely small transport of energy either by shock or thermal conduction away from the volume of interest. This offers extremely high precision machining with no heat or shock affected zones. For example, type 304 stainless steel, typically used in intravascular devices, such as stents, exhibits a thermal penetration depth of only 1.5 nm for a 100 femtosecond pulse compared to an optical penetration depth of approximately 5 nm. Furthermore, the lack of significant energy deposition beyond the volume of interest enables the use of high repetition (e.g., 1Hz-3MHz) lasers without the need for external cooling of the part being machined. Even though only a very small depth of material is removed from the workpiece per pulse, the high repetition rate enables extremely high cut rates (beyond 1 mm depth per second).

Cutting efficiency with these ultrashort pulses is significantly higher than that achievable for conventional long pulse lasers. This follows from two critical features: 1) there is little loss of energy away from the region of interest since thermal conduction during the pulse is negligible; and 2) there is no vaporization or transport of material during the pulse. With respect to the second of these features, during the laser pulse, there is insufficient time for hydrodymnamic expansion of the vaporized material. As a result, the laser pulse encounters the solid surface of the workpiece for the duration of the pulse, thereby depositing energy into the solid density material and raising a depth (dependent upon laser intensity and wavelength) to a temperature far beyond the boiling point. After the pulse is over, the depth which has been raised above the boiling point leaves the surface with an expansion velocity determined by the initial temperature. Typical temperatures in the expanding plasma are between 1 and 100 eV. The vaporized material is completely removed from the kerf before the arrival of the next laser pulse approximately 1 millisecond later. For example, an expanding vapor with even a low expansion velocity of $10^5$ cm/sec will be 1 meter away from the surface before the arrival of the next pulse. With conventional nanosecond or microsecond lasers, by contrast, the vapor will evolve during the laser pulse. This has the deleterious effect of reducing the coupling of the laser light to the solid cutting surface since the incident laser light will be scattered and absorbed by the vapor. This problem is completely overcome by the use of the very short pulses of the present invention.

The laser system used in the methods according to exemplary embodiments of the present invention produces a pulsed output beam having a selectively variable output pulse duration from about 10 femtoseconds to over 100 picoseconds at a variable pulse repetition rate from approximately 1 Hz to over 3MHz. The energy per pulse obtainable from the laser system is variable from approximately 0.1 microjoule to over 10 millijoules deliverable in a beam having a spot size variable from about 3 microns to over 1 centimeter. These exemplary parameters are particularly Effective in ablating all types of material without regard to its material properties or absorption characteristics, and without regard to the optical regime (IR, visible or UV wavelengths) in which the laser operates.

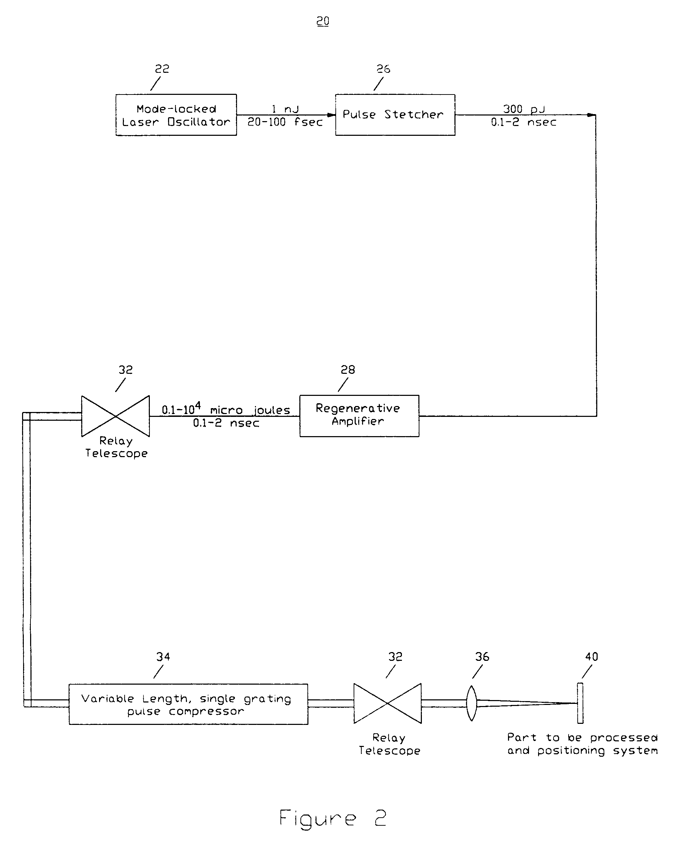

Although, as will be described in greater detail below, any type of laser system, capable of operating within the parameters described above, can be employed in practice of the invention, an exemplary embodiment of a suitable laser system is schematically depicted in Figure 2 and is described below. The laser system 20 has a mode-locked oscillator 22 that produces pulses of either the same or shorter duration than the final desired pulse duration. A commercially available oscillator producing 100 femtosecond pulses is adequate, although certain preferred embodiments of the invention use an oscillator producing 20 femtosecond pulses. Both of the exemplary oscillators 22 utilize titanium-doped sapphire as the laser material and the Kerr effect for mode-locking.

The pulses produced from the oscillator 24 are very low in energy, on the order of 1 nanojoule. These low energy pulses are stretched in the time prior to amplification by a factor of over one thousand using a pulse stretcher 26. The pulse stretcher 26 in the exemplary embodiment of the laser system 20 is a device employing a diffraction grating to disperse the various frequency components of the broad-bandwidth pulse arriving from the oscillator 22. By sending the various frequency components along different paths through an imaging telescope in the pulse stretcher 26, the pulse is lengthened in time by the amount $\Delta L/c$, where $\Delta L$ is the difference in optical path length between the various frequency components and c is the speed of light. The imaging telescope utilizes reflective optics (e.g., a parabolic mirror) to eliminate any unwanted dispersion that might be introduced by refractive optics.

The stretched pulse is amplified by several orders of magnitude to the millijoule level in the next stage of the laser system. Although many types of laser amplifiers could be used in this state, the preferred embodiment uses a regenerative amplifier 28. A regenerative amplifier is a device in which a pulse can make multiple passes through a single amplifier media. An exemplary embodiment of the regenerative amplifier 28 used in the system 20 employs titanium-doped sapphire as the gain medium of the regenerative amplifier 28.

As a result of the short storage time of Ti:sapphire, a second laser (not shown) is used to pump the Ti:sapphire gain medium. In the exemplary embodiment, this pump laser 30 is a frequency-doubled, Q-switched Neodymium-yttrium-aluminum-garnet (Nd:YAG) laser. The energy required to pump the Ti:sapphire regenerative amplifier 28 is typically greater than five times the energy output of the regenerative amplifier 28. The repetition rate of the laser system 20 is determined by the repetition rate of the pump laser. Switching of the pulse into and out of the regenerative amplifier 28 is accomplished with pulse switching technology based on the Pockels or acousto-optics effects, employing a Pockels cell, for example. The pulse is switched out of the regenerative amplifier 28, either before or at the point of saturation. Switchout after saturation actually reduces the pulse energy since subsequent passes in the cavity experience a loss which is greater than the single pass gain.

The regenerative amplifier 28 produces pulses up to 10 mJ in energy. Following amplification, the laser pulse is compressed by a variable length pulse compressor 34 employing a diffraction grating. Like the pulse stretcher 26, pulse compression occurs by controlling the optical path of the various frequency components of the laser pulse through the pulse compressor 34. Different frequency components are directed along differing optical paths by the angular dispersion of the grating. By controlling the dispersive path length taken by the various frequency components, a variable duration output pulse is obtained. With the exemplary, laser system 20 as above-described, a final pulse duration is obtainable which can be typically be adjusted between 0.02 and 100 picoseconds. The diffraction gratings in certain embodiments of the invention have densities as low as approximately 600 lines/mm and as high as approximately 1800 lines/mm. The diffraction gratings are standard holographic or ruled gratings. The pulse energy exiting the pulse compressor 34 is reduced by approximately 30 percent from that exiting the regenerative amplifier 28 as a result of the diffraction efficiency of the grating.

Following compression, the laser pulse is delivered to a focusing system 36 by either an open beam transport system, an articulated arm, an optical fiber or hollow core waveguide. In certain embodiments, this delivery provides additional compression of the pulse duration. If an open beam transport system is used, the beam transport comprises standard relay telescopes which are well known in the art, such as depicted at 32.

The focusing system 36 comprises either a simple or compound lens or Cassegranian-type mirror arrangement for focusing the pulse onto the target material of the workpiece 40 with the desired irradiance. The spot size is easily adjusted by moving the target away from best focus or by changing the focusing element. All of these focusing techniques are well known to those skilled in the art.

The laser system 20 can produce continuously tunable output from approximately 780 to over 1000 nanometers (nm) by a simple change of optics and minor adjustments to the angles of the gratings in the pulse stretcher and compressor. Operation at the second harmonic (400 to 500 nm) is accomplished by passing the beam through a thin nonlinear crystal (e.g., potassium dihydrogen phosphate (KDP), lithium borate, etc.) after compression. The crystal is cut for type I phase matching and is between 0.5 and 4 millimeters in length.

Under control of a computer (not shown), the laser system 20 makes the cuts on the workpiece 40 to create the intravascular device, such as the stent depicted in Figure 1. Alternatively, the device holding the workpiece 40 may be manipulated by the computer to move the workpiece in the path of the laser beam to create the cuts in the workpiece 40.

Although the present invention has been described with the example of a stent, the invention is also applicable to other intravascular devices, such as blood clot filters, occlusion devices, etc.

In addition to the disclosed method of cutting an intravascular device or stent from a hollow tubular workpiece, the present invention may be used to create an intravascular device starting from different shaped workpieces. For example, the workpiece may be a flat sheet that is cut according to a pre-defined pattern and then formed appropriately into a hollow cylindrical shape. Alternatively, rings can be cut from a tube and joined together to create the final intravascular device.

In the method of the present invention, a laser system that provides laser pulses of extremely short duration is used to make cuts on a workpiece to form an intravascular device. This creates an intravascular device which does not have any zones that have been subjected to heat or shock and have been damaged thereby. Hence, there is no possibility for the features of interest in the intravascular device being destroyed due to thermal excursion or shock caused by the laser cutting during processing of the intravascular device.

Although the present invention has been described and illustrated in detail, it is clearly understood that the same is by way of illustration and example only and is not to be taken by way of limitation, the spirit and scope of the present invention being limited only by the terms of the appended claims.

**Claims**

1. A method of forming an intravascular device or stent, comprising the steps of:

   positioning a workpiece for exposure to laser pulses; and
   subjecting the workpiece to laser pulses, each laser pulse having a duration of less than approximately 100 picoseconds, to selectively remove material from the workpiece to form the intravascular device or stent.

2. The method of Claim 1, wherein the intravascular device or stent is a stent, the method further comprising the step of directing the laser pulses in a pattern over the workpiece to form the stent.

3. The method of Claim 2, wherein the step of directing the laser pulses includes directing the laser pulses to selectively create open spaces in the workpiece.

4. The method of Claim 2, wherein the step of directing the laser pulses includes the step of moving the workpiece while the laser pulses are directed at the workpiece.

5. The method of Claim 1, wherein the step of subjecting the workpiece to laser pulses includes the step of generating laser pulses with an energy per pulse between approximately 0.1 microjoule to approximately 10 millijoules.

6. The method of Claim 5, wherein the step of subjecting the workpiece to laser pulses further includes the step of generating the laser pulses at a repetition rate between approximately 1 Hz to approximately 3 MHz.

7. The method of Claim 6, wherein the step of subjecting the workpiece to laser pulses further includes the step of focusing the laser pulses in a beam having a spot size between approximately 3 microns to approximately 1 centimeter.

8. A method of manufacturing an intravascular device or stent, comprising the steps of:

   positioning a workpiece for exposure to a laser beam; and
   directing a laser beam at the workpiece to remove at least one volume of material from a site on the workpiece with minimal transport of energy to the areas of the workpiece surrounding the volume of material.

9. The method of Claim 8, wherein the laser beam comprises laser pulses, the method further comprising the step of adjusting the pulse duration of the individual laser pulses such that a thermal penetration depth of the workpiece material during the pulse is on the order of an optical penetration depth of the workpiece material.

10. The method of Claim 9, wherein the step of adjusting includes adjusting the pulse duration to be less than 100 picoseconds.

11. The method of Claim 8, wherein the step of directing a laser beam includes changing the position of the workpiece relative to the laser beam to remove material from the workpiece in a pattern to form the intravascular device or stent.

12. The method of Claim 8, further comprising the step of removing at least two volumes of material wherein the material remaining between said at least two volumes is not greater than 0.002" at the narrowest region of the material between the at least two volumes.

13. An intravascular device or stent comprising :

material areas defining a pattern of material;
spaces from which volumes of material have been removed from the intravascular device or stent; and
the volumes of material being removed by directing laser pulses having a pulse duration less than 100 picoseconds at the intravascular device or stent to create the pattern of material without transferring significant energy to the material areas creating the pattern of material.

14. The intravascular device or stent of Claim 13, wherein the pattern of material is a substantially cylindrical hollow tube.

15. The intravascular device or stent of Claim 14 wherein the tube has a mesh of interconnected material with the spaces formed between the material in interstices of the mesh.

16. The intravascular device of stent of Claim 13, wherein the intravascular device or stent is made of stainless steel.

17. The intravascular device or stent of Claim 13, wherein the intravascular device or stent is made of shape-memory alloy.

18. The intravascular device or stent of Claim 13, wherein the pattern of material is a substantially planar sheet, wherein the substantially planar sheet is formed into a substantially cylindrical shape after removal of the volumes of material.

Figure 1a

Figure 1b

20

22

Mode-locked
Laser Oscillator

1 nJ
20-100 fsec

26

Pulse Stetcher

300 pJ
0.1-2 nsec

32

Relay
Telescope

0.1-10$^4$ micro joules
0.1-2 nsec

28

Regenerative
Amplifier

34

Variable Length, single grating
pulse compressor

32

Relay
Telescope

36

40

Part to be processed
and positioning system

Figure 2

EP 0 842 729 A1

| | European Patent Office | EUROPEAN SEARCH REPORT | Application Number EP 97 30 8358 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP 0 714 641 A (ADVANCED CARDIOVASCULAR SYSTEM) | 8,9,11, 13-16 | B23K26/00 A61F2/06 |
| A | * page 6, line 26 - line 32; figures 6-12 * | 1-7 | |
| A | US 5 262 614 A (KATAYAMA KAORU ET AL) | 1,8-10, 13 | |
| | * the whole document * | | |
| A,D | US 5 073 694 A (TESSIER JEFF M ET AL) | | |

TECHNICAL FIELDS SEARCHED (Int.Cl.6)

A61F
B23K

The present search report has been drawn up for all claims

| Place of search | Date of completion of the search | Examiner |
|---|---|---|
| THE HAGUE | 12 February 1998 | Aran, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

10